# EUROPEAN PATENT APPLICATION

(11) **EP 4 722 220 A1**
(43) Date of publication of application: **08.04.2026**
(21) Application number: 24815556.6
(22) Date of filing: 30.05.2024
(51) Int. Cl.: C07D 499/04, A61K 31/43, A61P 31/04, C07D 499/12, C07D 499/44

(54) **METHOD FOR PRODUCING BETA-LACTAM COMPOUND**

(30) Priority: 31.05.2023 JP 2023090147
(71) Applicant: FUJIFILM Toyama Chemical Co., Ltd., Chuo-ku Tokyo 104-0031 (JP)
(72) Inventor: FUJIMOTO, Taisuke, Toyama-shi, Toyama 930-8508 (JP); WADA, Kenji, Ashigarakami-gun, Kanagawa 258-8577 (JP); MATSUMOTO, Hideki, Ashigarakami-gun, Kanagawa 258-8577 (JP); ARAI, Tsuyoshi, Ashigarakami-gun, Kanagawa 258-8577 (JP); SATO, Keiichi, Ashigarakami-gun, Kanagawa 258-8577 (JP); HARADA, Kei, Ashigarakami-gun, Kanagawa 258-8577 (JP)
(74) Representative: Hoffmann Eitle
(86) International application number: PCT/JP2024/019822
(87) International publication number: WO 2024/248075

(57) **Abstract**

A method of producing a β-lactam compound, comprising: introducing an aqueous dispersion (i) of a β-lactam compound (a) having an amino group, an aqueous solution (ii) of a basic compound, and an organic solution (iii) of a compound having an activated carboxy group into different flow paths respectively and causing the aqueous dispersion (i), the aqueous solution (ii) and the organic solution (iii) to flow in the different flow paths; dissolving the β-lactam compound (a) while a mixture of the liquid (i) and the liquid (ii) flows, to obtain an aqueous solution (M1); and causing the β-lactam compound (a) to react with the compound having an activated carboxy group while a mixture (M2) of the aqueous solution (M1) and the liquid (iii) flows, to obtain a β-lactam compound (b) having an amide bond formed.

## Description

### FIELD OF THE INVENTION

The present invention relates to a method of producing a β-lactam compound.

### BACKGROUND OF THE INVENTION

A β-lactam compound (compound having a β-lactam ring) is widely known as an antibiotic or the like, and is a compound important for maintaining health of humans and other animals. For example, piperacillin (abbreviation: PIPC), which is a penicillin-based anti-bacterial drug, has a strong antibacterial activity, and has a wide antibacterial spectrum by being combined with tazobactam, which is a β-lactamase inhibitor. Tazobactam sodium-piperacillin sodium is designated as "pharmaceuticals requiring stable supply that give top priority to efforts" defined by the Ministry of Health, Labour and Welfare in Japan, and is also designated as "specified critical products (antimicrobial substance preparations)" based on the Economic Security Promotion Act. PIPC is chemically synthesized by using ampicillin (abbreviation: ABPC), which is a β-lactam-based amine compound, and 4-ethyl-2,3-dioxopiperazine-1-carbonyl chloride (hereinafter, also referred to as compound A) having an activated carboxy group as raw materials. An example of this reaction scheme is shown below. Et represents an ethyl group, and Ph represents a phenyl group.

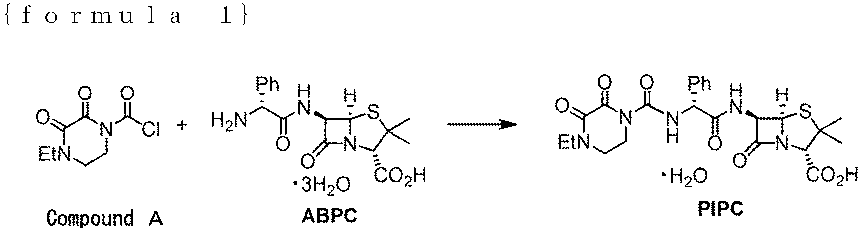

In the above reaction scheme, ABPC is dissolved in water to prepare an aqueous solution, the compound A is dissolved in an organic solvent to prepare an organic solution, and then reaction is performed in a two-phase system of an aqueous phase and an organic phase in the presence of a basic reagent (also referred to as a basic compound), whereby PIPC can be obtained in the aqueous phase. ABPC has low solubility in water, but the solubility in water is increased by adding a basic reagent. Therefore, the basic reagent not only acts as a neutralizing agent for hydrochloric acid generated in the reaction, but also is used as a dissolution accelerator when ABPC is made into an aqueous solution.

There is an attempt to carry out a reaction of obtaining an amide compound by reacting an amine compound with a compound having an activated carboxy group, such as the synthesis reaction of the PIPC, by a flow reaction which is a continuous synthesis method instead of a general batch-type reaction. For example, Patent Literature 1 describes the following invention.

A method of producing an amide compound using a flow-type reactor,
the flow-type reactor including:
a first flow path;
a second flow path;
a first mixing unit provided at a mixing section of the first flow path and the second flow path; and
a third flow path connected to the first mixing unit and disposed on a downstream side of the first mixing unit,
the method including:
   a mixing step of causing a first liquid containing a carboxylic acid halide to flow in the first flow path, causing a second liquid containing an amine compound having a molecular weight of 1,000 or less, an inorganic alkali, and water to flow in the second flow path, and mixing the first liquid and the second liquid in the first mixing unit to obtain a mixed liquid; and
   a reaction step of causing the mixed liquid to flow in the third flow path to react the carboxylic acid halide and the amine compound with each other in the third flow path, thus obtaining an amide compound.

According to the method of producing an amide compound described in Patent Literature 1, various carboxylic acid halides can be used, and a desired amide compound can be produced in a yield higher than that of a batch-type reaction by suppressing side reactions.

### CITATION LIST

### PATENT LITERATURES

Patent Literature 1: JP-A-2020-138961 ("JP-A" means an unexamined published Japanese patent application)

### SUMMARY OF THE INVENTION

### TECHNICAL PROBLEM

As a result of repeated studies by the present inventors, the present inventors have found that side reactions are likely to occur even in the synthesis reaction of PIPC when this reaction is performed by a batch-type. Although the mechanism of these side reactions is not necessarily clear, for example, as shown below, it is considered that a ring-opening reaction of ABPC due to contact between ABPC and a basic reagent for a long time, and an excessive reaction due to contact between PIPC as a target product and raw materials for a long time occur.

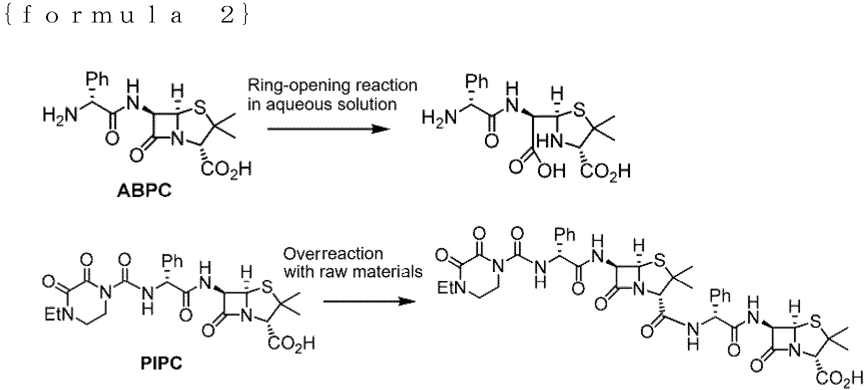

The present inventors have studied to solve the side reaction by applying a flow reaction. That is, studies have been made on a method of reacting an aqueous solution of a β-lactam compound (β-lactam compound having an amino group) which is an amine compound with an organic solution of a compound having an activated carboxy group in the presence of a basic reagent by a flow reaction in a two-phase reaction system, thereby more efficiently obtaining a target β-lactam compound having an amide bond formed by the reaction between the amino group and the activated carboxy group. However, even when the flow reaction is applied, there are limitations in improving the conversion rate of the target β-lactam compound. The present inventors have found that it is difficult to achieve a conversion rate exceeding 80% in the conversion rate based on the area percentage method by high performance liquid chromatography, for example.

The present invention provides a method of producing a β-lactam compound (b), the method comprising reacting a β-lactam compound (a) having an amino group with a compound having an activated carboxy group by a flow reaction, to efficiently obtain a β-lactam compound (b) having an amide bond formed by the reaction between the amino group and the activated carboxy group.

### SOLUTION TO PROBLEM

As a result of intensive studies in view of the above problems, the present inventors have found that the β-lactam compound (a) having an amino group in a state of an aqueous solution is likely to be decomposed, and this is one factor that causes a limitation in improving the conversion rate of the target β-lactam compound (b) even when a flow reaction is applied. Therefore, the present inventors have found that when the β-lactam compound (a) having an amino group is introduced into the flow path of the flow reaction system, the β-lactam compound (a) is introduced not in a state of an aqueous solution but in a state of an aqueous dispersion (slurry); while the aqueous dispersion flows in the flow path, the β-lactam compound (a) is mixed with a basic compound to increase the solubility of the β-lactam compound (a) having an amino group and form an aqueous solution; and the aqueous solution then joins an organic solution of an activated carboxylic acid compound to cause a flow reaction, and as a result, the conversion rate of the target β-lactam compound (b) is remarkably increased.

The present invention has been realized by conducting intensive research based on such knowledge.

The problems of the present invention are solved by the following means.
[1] A method of producing a β-lactam compound, including reacting a β-lactam compound (a) having an amino group with a compound having an activated carboxy group to obtain a β-lactam compound (b) having an amide bond formed by a reaction between the amino group and the activated carboxy group,
   the production method including:
   introducing a liquid (i) containing the β-lactam compound (a) dispersed in water, a liquid (ii) containing a basic compound dissolved in water, and a liquid (iii) containing the compound having an activated carboxy group dissolved in an organic solvent into different flow paths respectively, thereby causing each liquid to flow in the different flow paths;
   causing the liquid (i) and the liquid (ii) to join together to dissolve the β-lactam compound (a) and form an aqueous solution (M1) while a mixture flows downstream; and
   causing the aqueous solution (M1) and the liquid (iii) to join together to generate a mixture (M2) followed by, while a mixture (M2) flows downstream in a reaction flow path, causing the β-lactam compound (a) to react with the compound having an activated carboxy group in a two-phase system of an aqueous phase/an organic phase to obtain the β-lactam compound (b) in the aqueous phase.
[2] The production method described in [1], wherein the compound having an activated carboxy group is a carboxylic acid chloride compound.
[3] The production method described in [2], wherein the carboxylic acid chloride compound is 4-ethyl-2,3-dioxopiperazine-1-carbonyl chloride.
[4] The production method described in any one of [1] to [3], wherein the β-lactam compound (a) is ampicillin or 6-aminopenicillanic acid.
[5] The production method described in any one of [1] to [4], wherein the liquid (i) contains a basic compound.
[6] The production method described in [5], wherein the basic compound contained in the liquid (i) is an amine compound.

A numerical value range indicated by using the term "to" in the present specification means a range including the numerical values described before and after the term "to" as the lower limit value and the upper limit value, respectively.

In the present specification, when the size of the inner cross section (equivalent diameter) of a flow path, a mixing section, a mixer, or the like is described, the above size refers to a size excluding a connection portion between flow paths, a connection portion between a flow path and a mixing section, and a connection portion between a flow path and a mixer. That is, the size of each of the connection portions is appropriately adjusted using a connection tube or the like so that the fluid flows from upstream to downstream in the connection portion.

### ADVANTAGEOUS EFFECTS OF INVENTION

According to the method of producing a β-lactam compound of the present invention, a β-lactam compound (a) having an amino group and a compound having an activated carboxy group are reacted by a flow reaction, thereby making it possible to efficiently obtain a target β-lactam compound (b) having an amide bond formed by the reaction between the amino group and the activated carboxy group.

### BRIEF DESCRIPTION OF DRAWINGS

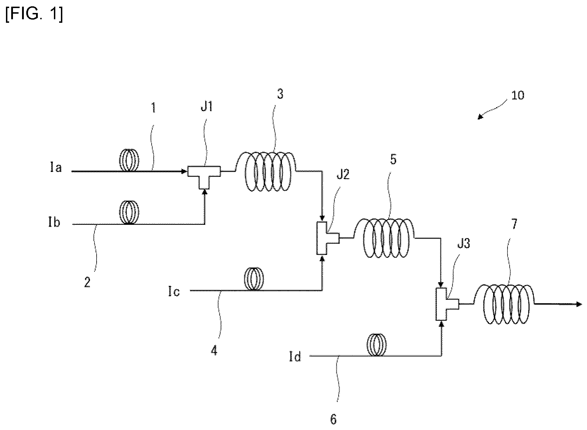

{FIG. 1}
FIG. 1 is an explanatory view showing an outline of an embodiment of a flow reaction system of the present invention.

### DESCRIPTION OF EMBODIMENTS

Preferable embodiments of the present invention will be described. The present invention is not limited to the following embodiments except as defined herein.

### [Production of β-lactam compound by flow reaction]

In the method of producing a β-lactam compound of the present invention (hereinafter, also referred to as the "production method of the present invention"), a flow reaction is employed.

In this flow reaction, a β-lactam compound (a) having an amino group is used as one raw material. The amino group of the β-lactam compound (a) does not constitute a cyclic amide group that characterizes the β-lactam structure. The amino group is usually an unsubstituted amino group. When the β-lactam compound (a) is introduced into the flow reaction system, the β-lactam compound (a) is in a state of an aqueous dispersion (slurry) in which the β-lactam compound (a) is dispersed in water. This aqueous dispersion of the β-lactam compound (a) is referred to as a liquid (i).

As the other raw material, a compound having an activated carboxy group (also referred to as an activated carboxylic acid compound) is used. When the activated carboxylic acid compound is introduced into the flow reaction system, the activated carboxylic acid compound is dissolved in an organic solvent to form an organic solution. This organic solution is referred to as a liquid (iii).

Furthermore, a basic compound is used as a neutralizing agent or a dissolution accelerator for the β-lactam compound (a). This basic compound is dissolved in water and introduced in a state of an aqueous solution into the flow reaction system. This aqueous solution is referred to as a liquid (ii).

In the liquids (i) and (ii), water as a medium may contain components other than water as long as the effects of the present invention are not impaired. Examples of the components other than water include water-soluble organic solvents compatible with water (for example, alcohol, acetone, N,N-dimethylacetamide, tetrahydrofuran, and the like). That is, in the present invention, the term "water" includes not only water itself but also a mixed liquid of water and a water-soluble organic solvent. The medium used for the liquids (i) and (ii) is preferably water (substantially free of components other than water). The water is preferably purified water, sterilized purified water, or water for injection, and more preferably sterilized purified water or water for injection.

The liquid (i) and the liquid (ii) are compatible with each other because the respective media are the same kind as each other or are mixed with each other. On the other hand, the liquid (iii) is incompatible with the liquids (i) and (ii). In the present invention, the term "compatible" means that liquid media dissolve each other to form a one-phase liquid (being mixed). On the other hand, the term "incompatible" means that the liquids do not substantially dissolve each other and are in a state of phase separation into two phases.

In the production method of the present invention, first, the liquids (i), (ii), and (iii) are introduced into different flow paths, and the respective liquids are allowed to flow in the respective flow paths. The liquid (i) and the liquid (ii) then join together at the downstream, and this allows the β-lactam compound (a) to be dissolved and brought into a state of an aqueous solution (M1) while the mixture flows downstream. Then, the aqueous solution (M1) and the liquid (iii) join together at the downstream, and the β-lactam compound (a) and the activated carboxylic acid compound react in a two-phase system of an aqueous phase/organic phase while the mixture (M2) flows downstream in the reaction flow path. Thus, the β-lactam compound (b) having an amide bond, which is a reaction product between the β-lactam compound (a) and the activated carboxylic acid compound, can be obtained in the aqueous phase.

In the present specification, the terms "upstream" and "downstream" are used with respect to the direction in which the liquid flows, and the side on which the liquid is introduced (the side on which the liquid flows) is the upstream and the side on which the liquid flows out is the downstream.

An embodiment of the flow reaction system used in the present invention will be described with reference to the drawing. Note that each drawing is an explanatory view for facilitating understanding of the present invention, and the size, the relative magnitude relationship, and the like of each component may be changed for convenience of description, and actual relationships are not illustrated as they are. Furthermore, the present invention is not limited to appearances and shapes illustrated in these drawings, except for the requirements defined by the present invention.

FIG. 1 is a schematic view showing an example of the flow reaction system used in the production method of the present invention. A flow reaction system (10) shown in FIG. 1 includes a flow path (1) including an inlet port (Ia) for introducing the liquid (i), a flow path (2) including an inlet port (Ib) for introducing the liquid (ii), a mixing section (J1) where the flow path (1) and the flow path (2) join together, a flow path (3) connected to the downstream end of the mixing section (J1), a flow path (4) including an inlet port (Ic) for introducing the liquid (iii), a mixing section (J2) where the flow path (3) and the flow path (4) join together, a reaction flow path (5) connected to the downstream end of the mixing section (J2), and a flow path (6) including an inlet port (Id) for introducing a reaction stop liquid, a mixing section (J3) where the flow path (5) and the flow path (6) join together, and a reaction stop flow path (7) connected to the downstream end of the mixing section (J3).

An aspect can be adopted in which a liquid feeding pump (not shown) such as a syringe pump or a diaphragm pump is normally connected to each of the inlet ports (Ia), (Ib), (Ic), and (Id), and respective liquids flow in respective flow paths at a desired flow rate by operating this pump.

Each configuration of the embodiment shown in FIG. 1 will be described in more detail.

### <Flow path (1)>

The flow path (1) is a flow path for supplying the liquid (i) introduced from the inlet port (Ia) to the mixing section (J1). The flow path (1) preferably has an equivalent diameter of 0.2 to 50 mm. When the equivalent diameter of the flow path (1) is set to 0.2 mm or more, an increase in pressure at the time of liquid feeding can be suppressed, and blockage of the flow path can be suppressed even when an insoluble matter is generated. When the equivalent diameter of the flow path (1) is set to 50 mm or less, the liquid temperature at the time of introduction into the mixing section (J1) can be appropriately controlled. The equivalent diameter of the flow path (1) is more preferably 0.5 to 30 mm, and still more preferably 1 to 20 mm.

The "equivalent diameter" is also referred to as an equilibrium diameter, and is a term used in the field of mechanical engineering. Assuming that a circular tube equivalent to a pipe line or a flow path having a given inner cross-sectional shape, the diameter of the inner cross section of the equivalent circular tube is referred to as an equivalent diameter. The equivalent diameter (deq) is defined as deq = 4A/p where A represents an inner cross-sectional area of a pipe line, and p represents a wetted perimeter (inner perimeter) of a pipe line. When the above definition is applied to a circular tube, this equivalent diameter corresponds to the diameter of the inner cross section of the circular tube. The equivalent diameter is used to estimate the flow characteristics or heat transfer characteristics of the pipe line based on the data of the equivalent circular tube, and represents the spatial scale (representative length) of a phenomenon. The equivalent diameter is deq = 4a²/4a = a for a square tube where a represents one side of the inner cross section of the tube, deq = a/3^{1/2} for an equilateral triangular tube where a represents one side of the inner cross section of the tube, and deq = 2h for a flow between parallel flat plates where h represents the height of the flow path (see, for example, "Mechanical Engineering Dictionary" edited by The Japan Society of Mechanical Engineers, 1997, Maruzen Co., Ltd.).

The length of the flow path (1) is not particularly limited, and the flow path (1) can be formed of, for example, a tube having a length of about 10 cm to 200 m (preferably 30 cm to 100 m).

The material of the tube is not particularly limited, and examples thereof include perfluoroalkoxy alkane (hereinafter, referred to as PFA), polytetrafluoroethylene (hereinafter, referred to as PTFE), aromatic polyether ketone-based resin, stainless steel, copper or a copper alloy, nickel or a nickel alloy, titanium or a titanium alloy, quartz glass, and soda lime glass. From the viewpoint of flexibility and chemical resistance, the material of the tube is preferably PFA, PTFE, stainless steel, a nickel alloy, or titanium.

The flow rate at which the liquid (i) is introduced from the inlet port (Ia) is not particularly limited, and can be appropriately set according to the purpose in consideration of the equivalent diameter of each flow path, the concentration and introduction volume flow rate of each liquid, and the like. For example, the flow rate is preferably 0.1 to 10,000 mL/min, more preferably 0.5 to 8000 mL/min, and still more preferably 1 to 6000 mL/min.

### - Liquid (i): liquid obtained by dispersing β-lactam compound (a) having amino group in water -

The liquid (i) to be introduced into the flow path (1) is a dispersion liquid (slurry) obtained by dispersing the β-lactam compound (a) in water. That is, in the liquid (i), the β-lactam compound (a) is present in a dispersed state without being dissolved. As described above, water as a medium may contain a water-soluble organic solvent compatible with water.

The liquid (i) may be a dispersion liquid containing the β-lactam compound (a) and water, and it is also preferable that the liquid (i) contains one kind or two or more kinds of basic compounds in a small amount to such an extent that the β-lactam compound (a) is not dissolved. When the liquid (i) contains a small amount of a basic compound, the viscosity of the liquid (i) which is an aqueous dispersion can be reduced, and blockage of the flow path over time can be further suppressed.

When the liquid (i) contains a basic compound in addition to the β-lactam compound (a), the molar ratio of the β-lactam compound (a) to the basic compound (β-lactam compound (a)/basic compound) is preferably 1000/1 to 5/1, more preferably 500/1 to 10/1, still more preferably 100/1 to 10/1, and even more preferably 50/1 to 20/1.

When the liquid (i) contains a basic compound in addition to the β-lactam compound (a), the pH of the liquid (i) is preferably 5 to 10, more preferably 6 to 9, and still more preferably 7 to 8 at 25°C. For example, as the basic compound to be added to the liquid (i), an amine compound (an amine compound other than the β-lactam compound (a)) is preferable, and a tertiary amine compound is more preferable. This basic compound is water-soluble, and is preferably an organic compound.

Examples of the basic compound include organic bases such as pyridine, diethylamine, dimethylaminopyridine, trimethylamine, triethylamine, and tripropylamine; and inorganic bases such as sodium hydroxide, potassium hydroxide, sodium hydrogen carbonate, potassium carbonate, and sodium carbonate, and one kind or two or more kinds thereof can be contained in the liquid (i). Among them, an organic base is preferable as the basic compound. Specifically, one selected from triethylamine, trimethylamine, sodium hydrogen carbonate, and sodium carbonate is preferable, and triethylamine is more preferable.

The β-lactam compound (a) is not particularly limited as long as it is a β-lactam compound having an amino group. Examples of the β-lactam compound (a) include 6-aminopenicillanic acid, 7-aminocephalosporanic acid, and ampicillin. Examples of the ampicillin include ampicillin hydrates and ampicillin anhydrides. The ampicillin hydrate is preferably ampicillin trihydrate. The β-lactam compound (a) is more preferably ampicillin trihydrate. When, for example, ampicillin trihydrate is used as the β-lactam compound (a), piperacillin can be obtained as the β-lactam compound (b). The β-lactam compound (a) contained in the liquid (i) is usually one kind.

The content of the β-lactam compound (a) in the liquid (i) is not particularly limited, and is appropriately adjusted in consideration of the introduction volume flow rate of the liquid (i), the concentration and introduction volume flow rate of each liquid, and the like. The content of the β-lactam compound (a) in the liquid (i) can be, for example, 0.01 to 10 M (mol/liter), and is preferably 0.02 to 5 M, more preferably 0.04 to 3 M, still more preferably 0.08 to 2 M, still more preferably 0.15 to 1.5 M, still more preferably 0.2 to 1.2 M, and still more preferably 0.3 to 1.0 M.

The temperature of the flow path (1) is not particularly limited as long as the liquid concentration (reaction temperature) in the section from the mixing section (J2) to the reaction flow path (5) can be controlled to a desired temperature. The temperature is usually set to a temperature that is not much different from the reaction temperature. For example, the temperature can be set to 0 to 40°C, and is preferably set to 0 to 30°C, more preferably set to 2 to 25°C, still more preferably set to 2 to 20°C, still more preferably set to 2 to 15°C, and still more preferably set to 2 to 10°C.

### <Flow path (2)>

The flow path (2) is a flow path for supplying the liquid (ii) introduced from the inlet port (Ib) to the mixing section (J1). The flow path (2) preferably has an equivalent diameter of 0.2 to 50 mm. When the equivalent diameter of the flow path (2) is set to 0.2 mm or more, an increase in pressure at the time of liquid feeding can be suppressed, and blockage of the flow path can be suppressed even when an insoluble matter is generated. When the equivalent diameter of the flow path (2) is set to 50 mm or less, the liquid temperature at the time of introduction into the mixing section (J1) can be appropriately controlled. The equivalent diameter of the flow path (2) is more preferably 0.5 to 30 mm, and still more preferably 1 to 20 mm.

The length of the flow path (2) is not particularly limited, and the flow path (2) can be formed of, for example, a tube having a length of about 10 cm to 200 m (preferably 30 cm to 100 m).

The material of the tube is not particularly limited, and a tube made of the material exemplified in the flow path (1) can be used.

The flow rate at which the liquid (ii) is introduced from the inlet port (Ib) is not particularly limited, and can be appropriately set according to the purpose in consideration of the equivalent diameter of each flow path, the concentration and introduction volume flow rate of each liquid, and the like. For example, the flow rate is preferably 0.1 to 10,000 mL/min, more preferably 0.5 to 8000 mL/min, and still more preferably 1 to 6000 mL/min.

The relationship between the flow rate rB at which the liquid (ii) is introduced from the inlet port (Ib) and the flow rate rA at which the liquid (i) is introduced from the inlet port (Ia) is also not particularly limited, and can be appropriately set in consideration of the concentration of each solution and the like. For example, [flow rate rA]/[flow rate rB] can be set to 10/1 to 1/10, and [flow rate rA]/[flow rate rB] is preferably set to 5/1 to 1/5. In the present specification, the unit of the flow rate is mL/min.

### - Liquid (ii): liquid obtained by dissolving basic compound in water -

The liquid (ii) to be introduced into the flow path (2) is an aqueous solution obtained by dissolving a basic compound in water. As described above, water as a medium may contain a water-soluble organic solvent compatible with water.

The basic compound contained in the liquid (ii) has the same meaning as the basic compound that can be contained in the liquid (i), and the preferred range thereof is also the same. That is, the basic compound contained in the liquid (ii) is preferably an amine compound (amine compound other than the β-lactam compound (a)), and more preferably a tertiary amine compound. This basic compound is water-soluble, and is preferably an organic compound.

Examples of the basic compound include organic bases such as pyridine, diethylamine, dimethylaminopyridine, trimethylamine, triethylamine, and tripropylamine; and inorganic bases such as sodium hydroxide, potassium hydroxide, sodium hydrogen carbonate, potassium carbonate, and sodium carbonate, and one kind or two or more kinds thereof can be contained in the liquid (ii). Among them, the basic compound is more preferably triethylamine, trimethylamine, sodium carbonate, or sodium hydroxide, and particularly preferably triethylamine.

The pH of the liquid (ii) is preferably 8 to 14, more preferably 9 to 13, and still more preferably 10 to 12 at 25°C.

The content of the basic compound in the liquid (ii) is not particularly limited as long as the β-lactam compound in the liquid (i) can be dissolved in water by being joined with the liquid (i), and is appropriately adjusted in consideration of the introduction volume flow rate of the liquid (ii), the concentration and introduction volume flow rate of each liquid, and the like. The content of the basic compound in the liquid (ii) can be, for example, 0.01 to 10 M (mol/liter), and is preferably 0.02 to 5 M, more preferably 0.04 to 3 M, still more preferably 0.08 to 2 M, still more preferably 0.15 to 1.5 M, still more preferably 0.2 to 1.2 M, and still more preferably 0.3 to 1.0 M.

The temperature of the flow path (2) is not particularly limited as long as the liquid concentration (reaction temperature) in the section from the mixing section (J2) to the reaction flow path (5) can be controlled to a desired temperature. The temperature is usually set to a temperature that is not much different from the reaction temperature. For example, the temperature can be set to 0 to 40°C, and is preferably set to 0 to 30°C, more preferably set to 2 to 25°C, still more preferably set to 2 to 20°C, still more preferably set to 2 to 15°C, and still more preferably set to 2 to 10°C.

### <Mixing section (J1)>

The liquid (i) introduced into the flow path (1) joins the liquid (ii) flowing in the flow path (2) at the mixing section (J1). The mixing section (J1) serves as a mixer, and is not particularly limited as long as the mixing section (J1) can join the flow path (1) and the flow path (2) into one flow path and send out the mixture to the flow path (3) connected to the downstream end of the mixing section (J1).

In the embodiment of FIG. 1, a T-shaped connector having three connection ports is used as the mixing section (J1). The equivalent diameter of the flow path in the mixing section (J1) is preferably 0.2 to 30 mm from the viewpoint of further improving mixing performance.

The material of the mixing section (J1) is not particularly limited, and for example, a part made of a material such as PFA, PTFE, an aromatic polyether ketone-based resin, stainless steel, copper or a copper alloy, nickel or a nickel alloy, titanium or a titanium alloy, quartz glass, or soda lime glass can be used.

As the T-shaped connector, a commercially available product can be widely used, and for example, a cross connector manufactured by Upchurch Scientific, Union Cross manufactured by Swagelok Company, a four-way joint manufactured by EYELA, a SUS cross mixer manufactured by IDEX Corporation, and the like can be used. In the present invention, the mixing section (J1) is not limited to the T-shaped connector, and for example, a Y-shaped connector may be used. An aspect can be adopted in which the liquid (i) and/or the liquid (ii) may be introduced from a plurality of inlet ports using a connector having four or more connection ports.

### <Flow path (3)>

The liquid (i) and the liquid (ii) join together and are mixed at the mixing section (J1), then flow into the flow path (3), and the β-lactam compound (a) dispersed in the liquid (i) is dissolved in water by the action of the basic compound of the liquid (ii) while the mixture flows downstream in the flow path (3). That is, the flow path (3) is a flow path for producing an aqueous solution (M1) of the β-lactam compound (a) and supplying the aqueous solution (M1) to the mixing section (J2).

The form of the flow path (3) is not particularly limited, and a tube is usually used. A preferable material of the flow path (3) is the same as the preferable material of the flow path (1) described above. In addition, the flowing time (dissolution time of the β-lactam compound (a)) in the flow path (3) of the mixture which is joined at the mixing section (J1) can be adjusted by the equivalent diameter and length of the flow path (3), the setting of volume flow rate of the liquid feeding pump, and the like. The equivalent diameter of the flow path (3) is preferably 0.2 to 50 mm, more preferably 0.3 to 30 mm, still more preferably 0.5 to 20 mm, still more preferably 0.7 to 15 mm, and still more preferably 1 to 12 mm. The length of the flow path (3) is preferably 0.5 to 50 m, and more preferably 1 to 30 m. In order to more uniformly mix the liquid (i) and the liquid (ii) in the flow path (3) to more reliably obtain an aqueous solution of the β-lactam compound (a), it is preferable to dispose a static mixer in the middle of the flow path (3).

The flowing time of the mixture flowing in the flow path (3) is preferably 0.01 to 10 minutes, more preferably 0.02 to 5 minutes, and still more preferably 0.03 to 1 minutes.

In the mixture (aqueous solution (M1)) of the liquid (i) and the liquid (ii) flowing in the flow path (3), the content of the basic compound is preferably 0.30 to 0.60 M (mol/liter), more preferably 0.31 to 0.56 M, and still more preferably 0.33 to 0.50 M. In addition, the pH of this mixture (aqueous solution (M1)) is preferably 9 to 13 and more preferably 10 to 12 at 25°C.

In the mixture (aqueous solution (M1)), the content of the β-lactam compound (a) is preferably 0.14 to 0.27 M (mol/liter), more preferably 0.15 to 0.25 M, and still more preferably 0.16 to 0.24 M.

In the mixture (aqueous solution (M1)), the molar ratio of the β-lactam compound (a) to the basic compound (β-lactam compound (a)/basic compound) is preferably 1.0/1.6 to 1.0/3.0, more preferably 1.0/1.8 to 1.0/2.5, and still more preferably 1.0/2.0 to 1.0/2.3.

The temperatures of the mixing section (J1) and the flow path (3) are not particularly limited as long as the liquid concentration (reaction temperature) in the section from the mixing section (J2) to the reaction flow path (5) can be controlled to a desired temperature. The temperature is usually set to a temperature that is not much different from the reaction temperature. For example, the temperature can be set to 0 to 40°C, and is preferably set to 0 to 30°C, more preferably set to 2 to 25°C, still more preferably set to 2 to 20°C, still more preferably set to 2 to 15°C, and still more preferably set to 2 to 10°C.

### <Flow path (4)>

The flow path (4) is a flow path for supplying the liquid (iii) introduced from the inlet port (Ic) to the mixing section (J2). The flow path (4) preferably has an equivalent diameter of 0.2 to 50 mm. When the equivalent diameter of the flow path (4) is set to 0.2 mm or more, an increase in pressure at the time of liquid feeding can be suppressed, and blockage of the flow path can be suppressed even when an insoluble matter is generated. When the equivalent diameter of the flow path (4) is set to 50 mm or less, the liquid temperature at the time of introduction into the mixing section (M1) can be appropriately controlled. The equivalent diameter of the flow path (4) is more preferably 0.5 to 30 mm, and still more preferably 1 to 20 mm.

The length of the flow path (4) is not particularly limited, and the flow path (4) can be formed of, for example, a tube having a length of about 10 cm to 200 m (preferably 30 cm to 100 m).

The material of the tube is not particularly limited, and a tube made of the material exemplified in the flow path (1) can be used.

The flow rate at which the liquid (iii) is introduced from the inlet port (Ic) is not particularly limited, and can be appropriately set according to the purpose in consideration of the equivalent diameter of each flow path, the concentration and introduction volume flow rate of each liquid, and the like. For example, the flow rate is preferably 0.1 to 30,000 mL/min, more preferably 0.5 to 20,000 mL/min, and still more preferably 1 to 15000 mL/min.

The relationship between the flow rate rD at which the aqueous solution (M1) is introduced from the flow path (3) to the mixing section (J2) and the flow rate rC at which the liquid (iii) is introduced from the inlet port (Ic) is also not particularly limited, and can be appropriately set in consideration of the concentration of each solution and the like. For example, [flow rate rC]/[flow rate rD] can be set to 10/1 to 1/10, and [flow rate rC]/[flow rate rD] is preferably set to 5/1 to 1/5.

### - Liquid (iii): liquid obtained by dissolving activated carboxylic acid compound in organic solvent -

The liquid (iii) flowing in the flow path (4) is an organic solution obtained by dissolving an activated carboxylic acid compound in an organic solvent.

Examples of the organic solvent used in this reaction include ketone solvents such as 2-butanone and 4-methyl-2-pentanone; and ester solvents such as methyl acetate, ethyl acetate, propyl acetate and butyl acetate, and one kind or two or more kinds of these can be used. As the organic solvent, an ester solvent is preferable, ethyl acetate, propyl acetate, isopropyl acetate, and butyl acetate are more preferable, and ethyl acetate is still more preferable.

The activated carboxylic acid compound contained in the liquid (iii) is not particularly limited as long as it is a compound having an activated carboxy group. For example, carboxylic acid anhydrides and carboxylic acid halides (compounds having a structure of "-C(=O)-[halogen element]") are preferable, carboxylic acid chloride compounds (compounds having a structure of "-C(=O)-Cl") are more preferable, and 4-ethyl-2,3-dioxopiperazine-1-carbonyl chloride is still more preferable. One kind or two or more kinds of the activated carboxylic acid compound contained in the liquid (iii) may be used, and preferably, one kind of the activated carboxylic acid compound is used.

The content of the activated carboxylic acid compound in the liquid (iii) is not particularly limited, and is appropriately adjusted in consideration of the introduction volume flow rate of the liquid (iii), the concentration and introduction volume flow rate of each liquid, and the like. The content of the activated carboxylic acid compound in the liquid (iii) can be, for example, 0.01 to 10 M (mol/liter), and is preferably 0.02 to 5 M, more preferably 0.04 to 3 M, still more preferably 0.08 to 2 M, still more preferably 0.10 to 1.5 M, still more preferably 0.12 to 1.2 M, still more preferably 0.15 to 1.0 M, still more preferably 0.16 to 0.8 M, still more preferably 0.17 to 0.6 M, and still more preferably 0.18 to 0.4 M.

The temperature of the flow path (4) is not particularly limited as long as the liquid concentration (reaction temperature) in the section from the mixing section (J2) to the reaction flow path (5) can be controlled to a desired temperature. The temperature is usually set to a temperature that is not much different from the reaction temperature. For example, the temperature can be set to 0 to 40°C, and is preferably set to 0 to 30°C, more preferably set to 2 to 25°C, still more preferably set to 2 to 20°C, still more preferably set to 2 to 15°C, and still more preferably set to 2 to 10°C.

### <Mixing section (J2)>

The aqueous solution (M1) flowing in the flow path (3) and the liquid (iii) flowing in the flow path (4) join together at the mixing section (J2). The mixing section (J2) serves as a mixer, and is not particularly limited as long as the mixing section (J2) can join the flow path (3) and the flow path (4) into one flow path and send out the mixture to the reaction flow path (5) connected to the downstream end of the mixing section (J2).

In the embodiment of FIG. 1, a T-shaped connector having three connection ports is used as the mixing section (J2). The equivalent diameter of the flow path in the mixing section (J2) is preferably 0.2 to 30 mm from the viewpoint of further improving mixing performance.

For the material, shape, number of connection ports, and the like of the mixing section (J2), those described in the mixing section (J1) can be directly applied to the mixing section (J2).

In the mixture (uniform mixed liquid in an unreacted state) immediately after being joined at the mixing section (J2), the content of the activated carboxylic acid compound in the mixture is such that the amount of the activated carboxy group is preferably 0.80 to 1.20 equivalent, more preferably 0.85 to 1.15 equivalent, more preferably 0.90 to 1.10 equivalent, more preferably 0.93 to 1.07 equivalent, still more preferably 0.95 to 1.05 equivalent in terms of molar ratio with respect to the amount of the amino group of the β-lactam compound (a). Therefore, when the activated carboxylic acid compound has one activated carboxy group in one molecule and the β-lactam compound (a) has one amino group in one molecule, the molar ratio of the β-lactam compound (a) to the activated carboxylic acid compound (β-lactam compound (a)/activated carboxylic acid compound) in the mixture immediately after being joined at the mixing section (J2) is preferably 0.80 to 1.20, more preferably 0.85 to 1.15, still more preferably 0.90 to 1.10, still more preferably 0.93 to 1.07, still more preferably 0.95 to 1.05. In the present invention, when the activated carboxy group is an acetic anhydride structure (-C(=O)-O-C(=O)-), one amide bond is formed from one acetic anhydride structure, and thus one acetic anhydride structure is counted as one activated carboxy group.

### <Reaction flow path (5)>

The mixture, which is joined and mixed at the mixing section (J2), flows into the reaction flow path (5), and the β-lactam compound (a) and the activated carboxylic acid compound react to form an amide bond while the mixture flows downstream in the reaction flow path (5), and thus a target β-lactam compound (b) is produced. This reaction is a two-phase reaction system in which an aqueous phase and an organic phase are phase-separated. Usually, a two-phase reaction proceeds in a state in which an aqueous phase is a continuous phase and oil droplets are dispersed therein, or in a state in which an oil phase is a continuous phase and water droplets are dispersed therein, and a target β-lactam compound (b) is generated in the aqueous phase.

The form of the reaction flow path (5) is not particularly limited, and for example, a tube may be used. A preferable material of the reaction flow path (5) is the same as the preferable material of the flow path (1) described above. In addition, the reaction time can be adjusted by the equivalent diameter and length of the reaction flow path (5), the setting of volume flow rate of the liquid feeding pump, and the like. Usually, the equivalent diameter of the reaction flow path (5) is preferably 0.2 to 50 mm, more preferably 0.3 to 30 mm, still more preferably 0.5 to 20 mm, still more preferably 0.7 to 15 mm, and still more preferably 1 to 12 mm. The length of the reaction flow path (5) is preferably 0.5 to 50 m, and more preferably 1 to 30 m.

The flowing time of the mixture flowing in the reaction flow path (5) is preferably 0.005 to 10 minutes, more preferably 0.01 to 5 minutes, and still more preferably 0.015 to 1 minute.

The temperatures (that is, the reaction temperature) of the mixing section (J2) and the reaction flow path (5) can be, for example, 0 to 40°C, and are preferably 0 to 30°C, more preferably 2 to 25°C, still more preferably 2 to 20°C, still more preferably 2 to 15°C, and still more preferably 2 to 10°C.

The reaction liquid flowing in the reaction flow path (5) can be recovered as it is. Furthermore, for example, the reaction liquid flowing in the reaction flow path (5) can join a reaction stop liquid introduced from the inlet port (Id) at the mixing section (J3), and then the mixture is caused to flow downstream in the flow path (7) to be recovered. The aqueous phase is separated and recovered from the recovery liquid to obtain a target β-lactam compound (b) in the aqueous phase.

The form of the flow path (7) is not particularly limited, and for example, a tube is used. A preferable material, size, and the like of the flow path (7) are the same as the preferable material, size, and the like of the flow path (1). In addition, the form of the mixing section (J3) is also not particularly limited, and a preferable material, size, and the like of the mixing section (J3) are the same as the preferable material, size, and the like of the mixing section (J1).

As the reaction stop liquid, an aqueous solution of an acidic compound such as acetic acid or hydrochloric acid can be used. These can be used singly or in combination, and acetic acid or an aqueous solution of acetic acid is preferable, and an aqueous solution of acetic acid is more preferable.

The liquid separation operation for extracting the aqueous phase from the recovery liquid can be performed by an ordinary method. The recovered aqueous phase is subjected to crystallization, filtration, drying, and the like by an ordinary method, thereby making it possible to obtain a target crystal of the β-lactam compound (b).

The production method of the present invention is not limited to the embodiment shown in FIG. 1. For example, an aspect can be adopted in which the liquid (ii) is prepared in a flow reaction system and sent to the mixing section (J1). As an example, an aspect can be adopted in which a stock liquid (for example, triethylamine) of a basic compound and water are caused to flow in different flow paths, the two liquids are joined to prepare a liquid (ii), and the mixture is fed to the mixing section (J1).

The present invention will be described in more detail based on Examples. However, the present invention is not limited to the Examples.

### EXAMPLES

### [Construction of flow reaction system]

A reaction system was constructed by removing the flow path (6), the mixing section (J3), and the reaction stop flow path (7) from the flow reaction system 10 having the configuration shown in FIG. 1, and a β-lactam compound (b) was synthesized.

FIG. 1 is a schematic explanatory view, and specific reaction conditions are as follows.

### Liquid feeding pump (not shown):

The liquid (i) was introduced into the inlet port (Ia) using a Mohno pump. A Mohno pump (model: 3HMC025F) manufactured by HEISHIN Ltd. connected to a brushless motor (model: BMUD30-A2) manufactured by Oriental Motor Co., Ltd. was used. A stirring blade for stirring the liquid (i) as a slurry was installed above the filling tank of the Mohno pump, the liquid (i) was stirred at 300 rpm to prepare a uniform dispersion liquid, and this was introduced into the Mohno pump.

The liquids (ii) and (iii) were introduced into the inlet ports (Ib) and (Ic) using a syringe pump. A gastight syringe (Luer lock syringe manufactured by Hamilton Company Japan K.K., 100 mL) was used as a syringe pump.

### Temperature control:

The flow paths (3) and (4), the mixing section (J2), and the reaction flow path (5) were disposed in a thermostatic bath (small desktop low-temperature water bath CBi-270A manufactured by AS ONE Corporation) set at 5°C.

### Flow path (1):

A portion up to the discharge port of the Mohno pump was defined as the flow path (1).

### Flow path (2):

A PFA tube having an outer diameter of 1/16 inches, an inner diameter of 1.0 mm, and a length of 0.5 m was used as the flow path (2).

### Mixing section (J1)

Union T (SS-400-3) manufactured by Swagelock Company was used. The flow path (1) and the flow path (2) were orthogonally connected with a T-shaped connector of the mixing section (J1), and the remaining connection port was used as a discharge port of the mixing section (J1).

### Flow path (3):

A static mixer (model T3-27R-1TP manufactured by Noritake Co., Ltd.) having a length of 17 cm was connected to the mixing section (J1), and a PFA tube having an outer diameter of 1/8 inches, an inner diameter of 1.59 mm, and a length of 0.5 m and a SUS316 tube having an outer diameter of 1/16 inches, an inner diameter of 1.0 mm, and a length of 1 m were sequentially connected to an outlet of the static mixer to form a flow path (3).

### Flow path (4)

A PFA tube having an outer diameter of 1/16 inches, an inner diameter of 1.0 mm, and a length of 0.5 m and a PFA tube having an outer diameter of 1/8 inches, an inner diameter of 1.59 mm, and a length of 1 m were connected in this order to form a flow path (4).

### Mixing section (J2)

Union T (SS-400-3) manufactured by Swagelock Company was used. The flow path (3) and the flow path (4) were orthogonally connected with a T-shaped connector of the mixing section (J2), and the remaining connection port was used as a discharge port of the mixing section (J2).

### Reaction flow path (5)

A PFA tube having an outer diameter of 1/8 inches, an inner diameter of 1.59 mm, and a length of 1.0 m was used as the reaction flow path (5). A PFA tube having an outer diameter of 1/16 inches, an inner diameter of 1.0 mm, and a length of 0.5 m was connected to the outlet of the reaction flow path (5), and the reaction liquid was recovered into the flask from the outlet of the PFA tube.

### [Determination of conversion rate based on area percentage method by high performance liquid chromatography]

The measurement conditions of high performance liquid chromatography (HPLC) were as follows.
Apparatus: Prominence series (manufactured by Shimadzu Corporation)
Detector: UV/VIS detector SPD-20A, 254 nm
Column: ATLANTIS T3 3 µL, 4.6 × 150 mm (manufactured by Waters Corporation)
Column oven temperature: 30°C
Mobile phase A: water, 0.1% phosphoric acid
Mobile phase B: acetonitrile, 0.1% phosphoric acid
Mobile phase concentration gradient:
   mobile phase B: 10% → 80% (0 minutes → 10 minutes)
   mobile phase B: 80% (10 minutes → 15 minutes)
   Volume flow rate: 1.0 mL/min
   Sample concentration: reaction liquid was diluted 20 times with water/acetonitrile (1/1)
   Sample injection amount: 1 µL
   Data collection time: 0 to 15 minutes after sample injection

### [Example 1]

In 42 g of water, 10.0 g of ampicillin trihydrate was dispersed to make a total amount of 50 mL, and this was used as a liquid (i). In addition, 10.0 g of triethylamine was dissolved in cold water to make a total amount of 200 mL, and this was used as a liquid (ii). Furthermore, 4.2 g of 4-ethyl-2,3-dioxopiperazine-1-carbonyl chloride was dissolved in ethyl acetate to make a total amount of 100 mL, and this was used as a liquid (iii). The liquid (i) was fed at a flow rate of 16 mL/min, the liquid (ii) was fed at a flow rate of 32 mL/min, and the liquid (iii) was fed at a flow rate of 38 mL/min. After the liquid feeding state was stabilized, the reaction liquid (liquid containing the reaction product) was recovered in a flask for 20 seconds, and 17.3 g of an aqueous phase was obtained by liquid separation operation. The resulting aqueous phase was analyzed by HPLC, and the result showed that the conversion rate of the target product (β-lactam compound (b)) as determined by an area percentage method was 97.3%.

To the resulting aqueous phase, 0.4 mL of acetic acid at a concentration of 10 mass% and seed crystals of piperacillin hydrate were added, and the mixture was stirred for 1 hour. Subsequently, 4 mol/L hydrochloric acid was added to the mixture until the pH became 2 or less. The resulting crystals were filtered, washed with a small amount of water and air-dried to obtain 1.2 g of piperacillin hydrate (yield 85%).

### [Example 2]

In Example 1, 5.4 g of 6-aminopenicillanic acid was dispersed in water instead of ampicillin trihydrate, and 0.17 mL of triethylamineamine was added to prepare a total amount of 50 mL of an aqueous dispersion. A reaction was performed for 15 seconds under the same conditions as in Example 1 except for the above, and 12.8 g of an aqueous phase was obtained. The resulting aqueous phase was analyzed by HPLC, and the result showed that the conversion rate of a target product as determined by an area percentage method was 84.0%.

### [Example 3]

The same procedure as in Example 1 was performed except that in Example 1, 2.1 g of acetic anhydride was used instead of 4-ethyl-2,3-dioxopiperazine-1-carbonyl chloride, thereby obtaining 17.1 g of an aqueous phase. The resulting aqueous phase was analyzed by HPLC, and the result showed that the conversion rate of a target product as determined by an area percentage method was 82.5%.

### [Example 4]

The same procedure as in Example 1 was performed except that in Example 1, 0.17 mL of triethylamine was added to an aqueous dispersion of 10.0 g of ampicillin trihydrate to prepare a total amount of 50 mL of an aqueous dispersion, thereby obtaining 17.4 g of an aqueous phase. The resulting aqueous phase was analyzed by HPLC, and the result showed that the conversion rate of a target product as determined by an area percentage method was 96.3%.

### [Example 5]

The same procedure as in Example 1 was performed except that in Example 1, 0.10 g of sodium hydrogen carbonate was added to an aqueous dispersion of 10.0 g of ampicillin trihydrate to prepare a total amount of 50 mL of an aqueous dispersion, thereby obtaining 16.6 g of an aqueous phase. The resulting aqueous phase was analyzed by HPLC, and the result showed that the conversion rate of a target product as determined by an area percentage method was 97.0%.

### [Example 6]

The same procedure as in Example 1 was performed except that in Example 1, the flow rate of the syringe pump feeding the liquid (ii) was 40 mL/min, and the time for recovering the reaction liquid (liquid containing the reaction product) in the flask was 15 seconds, thereby obtaining 13.4 g of an aqueous phase. The resulting aqueous phase was analyzed by HPLC, and the result showed that the conversion rate of a target product as determined by an area percentage method was 97.7%.

### [Example 7]

In 42 g of water, 10.0 g of ampicillin trihydrate was dispersed to make a total amount of 50 mL, and this was used as a liquid (i). In addition, 9.0 g of triethylamine was dissolved in cold water to make a total amount of 100 mL, and this was used as a liquid (ii). Furthermore, 4.2 g of 4-ethyl-2,3-dioxopiperazine-1-carbonyl chloride was dissolved in ethyl acetate to make a total amount of 100 mL, and this was used as a liquid (iii). The liquid (i) was fed at a flow rate of 16 mL/min, the liquid (ii) was fed at a flow rate of 19 mL/min, and the liquid (iii) was fed at a flow rate of 38 mL/min. After the liquid feeding state was stabilized, the reaction liquid (liquid containing the reaction product) was recovered in the flask for 30 seconds, and 19.8 g of an aqueous phase was obtained by liquid separation operation. The resulting aqueous phase was analyzed by HPLC, and the result showed that the conversion rate of a target product as determined by an area percentage method was 96.1%.

To the resulting aqueous phase, 0.6 mL of 10% acetic acid and seed crystals of piperacillin hydrate were added, and the mixture was stirred for 1 hour. Subsequently, 4 mol/L hydrochloric acid was added to the mixture until the pH became 2 or less. The resulting crystals were filtered, washed with a small amount of water and air-dried to obtain 1.78 g of piperacillin hydrate (yield 84%).

### [Example 8]

The same procedure as in Example 7 was performed except that in Example 7, 0.17 mL of triethylamine was added to an aqueous dispersion of 10.0 g of ampicillin trihydrate to prepare a total amount of 50 mL of an aqueous dispersion, thereby obtaining 19.1 g of an aqueous phase. The resulting aqueous phase was analyzed by HPLC, and the result showed that the conversion rate of a target product as determined by an area percentage method was 94.7%.

To the resulting aqueous phase, 0.6 mL of 10% acetic acid and seed crystals of piperacillin hydrate were added, and the mixture was stirred for 1 hour. Subsequently, 4 mol/L hydrochloric acid was added to the mixture until the pH became 2 or less. The resulting crystals were filtered, washed with a small amount of water and air-dried to obtain 1.76 g of piperacillin hydrate (yield 83%).

### [Example 9]

The same procedure as in Example 1 was performed except that in Example 1, the temperature of the thermostatic bath was 20°C (the reaction temperature was 20°C), thereby obtaining 17.1 g of an aqueous phase. The resulting aqueous phase was analyzed by HPLC, and the result showed that the conversion rate of a target product as determined by an area percentage method was 97.1%.

### [Example 10]

The same procedure as in Example 1 was performed except that in Example 1, 0.68 mL of triethylamine was added to an aqueous dispersion of 10.0 g of ampicillin trihydrate to prepare a total amount of 50 mL of an aqueous dispersion, thereby obtaining 17.2 g of an aqueous phase. The resulting aqueous phase was analyzed by HPLC, and the result showed that the conversion rate of a target product as determined by an area percentage method was 95.4%.

### [Example 11]

The same procedure as in Example 1 was performed except that in Example 1, 0.07 mL of triethylamine was added to an aqueous dispersion of 10.0 g of ampicillin trihydrate to prepare a total amount of 50 mL of an aqueous dispersion, thereby obtaining 17.6 g of an aqueous phase. The resulting aqueous phase was analyzed by HPLC, and the result showed that the conversion rate of a target product as determined by an area percentage method was 96.8%.

### [Example 12]

The same procedure as in Example 1 was performed except that in Example 1, 0.34 mL of triethylamine was added to an aqueous dispersion of 10.0 g of ampicillin trihydrate to prepare a total amount of 50 mL of an aqueous dispersion, thereby obtaining 17.3 g of an aqueous phase. The resulting aqueous phase was analyzed by HPLC, and the result showed that the conversion rate of a target product as determined by an area percentage method was 96.7%.

### [Example 13]

The same procedure as in Example 1 was performed except that in Example 1, the stirring in the filling tank of the Mohno pump was performed at 100 rpm, thereby obtaining 12.8 g of an aqueous phase. The resulting aqueous phase was analyzed by HPLC, and the result showed that the conversion rate of a target product as determined by an area percentage method was 98.0%.

### [Example 14]

The same procedure as in Example 4 was performed except that in Example 4, the stirring in the filling tank of the Mohno pump was performed at 100 rpm, thereby obtaining 12.3 g of an aqueous phase. The resulting aqueous phase was analyzed by HPLC, and the result showed that the conversion rate of a target product as determined by an area percentage method was 97.8%.

### [Example 15]

The same procedure as in Example 4 was performed except that in Example 4, the stirring in the filling tank of the Mohno pump was not performed, thereby obtaining 12.2 g of an aqueous phase. The resulting aqueous phase was analyzed by HPLC, and the result showed that the conversion rate of a target product as determined by an area percentage method was 94.9%.

### [Example 16]

The same procedure as in Example 4 was performed except that in Example 4, the stirring in the filling tank of the Mohno pump was continued for 1 hour, thereby obtaining 12.0 g of an aqueous phase. The resulting aqueous phase was analyzed by HPLC, and the result showed that the conversion rate of a target product as determined by an area percentage method was 95.6%.

### [Comparative Example 1]

In water, 10.0 g of ampicillin trihydrate was dispersed, and 6.3 g of triethylamine was then added to dissolve the ampicillin trihydrate, thereby obtaining a total amount of 50 mL of an aqueous solution (i-c). In addition, 4.2 g of 4-ethyl-2,3-dioxopiperazine-1-carbonyl chloride was dissolved in ethyl acetate to make a total amount of 100 mL, and this was used as a liquid (iii). Using a reaction system constructed by removing the flow path (1), the flow path (2), the mixing section (J1), the flow path (6), the mixing section (J3), and the reaction stop flow path (7) from the flow reaction system 10 having the configuration shown in FIG. 1, the aqueous solution (i-c) was fed into the flow path (3) at a flow rate of 16 mL/min, and the liquid (iii) was fed from the inlet port (Ic) at a flow rate of 38 mL/min in the same manner as described above. After the liquid feeding state was stabilized, the reaction liquid (liquid containing the reaction product) was recovered in the flask for 15 seconds, and 3.8 g of an aqueous phase was obtained by liquid separation operation. The resulting aqueous phase was analyzed by HPLC, and the result showed that the conversion rate of a target product as determined by an area percentage method was 70.3%.

Comparative Example 1 is different from each of the above Examples in that the β-lactam compound (a) having an amino group is supplied in a state of an aqueous solution instead of an aqueous dispersion (slurry). It can be seen that the conversion rate of the target β-lactam compound (b) in the form of Comparative Example 1 is much lower than that in Examples 1 to 15 described above.

### [Comparative Example 2]

In water, 10.0 g of ampicillin trihydrate was dispersed, and 5.0 g of triethylamine was then added to dissolve the ampicillin trihydrate, thereby obtaining a total amount of 50 mL of an aqueous solution (i-d), and this was left to stand at room temperature for 1 hour. In addition, 4.2 g of 4-ethyl-2,3-dioxopiperazine-1-carbonyl chloride was dissolved in ethyl acetate to make a total amount of 100 mL, and this was used as a liquid (iii). Using the same reaction system as in Comparative Example 1, the aqueous solution (i-d) was fed into the flow path (3) at a flow rate of 16 mL/min, and the liquid (iii) was fed from the inlet port (Ic) at a flow rate of 38 mL/min. After the liquid feeding state was stabilized, the reaction liquid (liquid containing the reaction product) was recovered in the flask for 15 seconds, and 4.2 g of an aqueous phase was obtained by liquid separation operation. The resulting aqueous phase was analyzed by HPLC, and the result showed that the conversion rate of a target product as determined by an area percentage method was 75.2%.

Having described the present invention as related to the embodiment, it is our intention that the present invention not be limited by any of the details of the description, unless otherwise specified, but rather be construed broadly within its spirit and scope as set out in the accompanying claims.

The present application claims priority of Patent Application No. 2023-090147 filed in Japan on May 31, 2023, which is herein incorporated by reference as part of the present specification.

### DESCRIPTION OF SYMBOLS

10 Flow reaction system
la, Ib, Ic, Id Inlet port
1, 2, 3, 4, 5, 6, 7 Flow path
J1, J2, J3 Mixing section

## Claims

1. A method of producing a β-lactam compound, comprising reacting a β-lactam compound (a) having an amino group with a compound having an activated carboxy group to obtain a β-lactam compound (b) having an amide bond formed by a reaction between the amino group and the activated carboxy group,
the production method comprising:
introducing a liquid (i) containing the β-lactam compound (a) dispersed in water, a liquid (ii) containing a basic compound dissolved in water, and a liquid (iii) containing the compound having an activated carboxy group dissolved in an organic solvent into different flow paths respectively, thereby causing each liquid to flow in the different flow paths;
causing the liquid (i) and the liquid (ii) to join together to dissolve the β-lactam compound (a) and form an aqueous solution (M1) while a mixture flows downstream; and
causing the aqueous solution (M1) and the liquid (iii) to join together to generate a mixture (M2) followed by, while the mixture (M2) flows downstream in a reaction flow path, causing the β-lactam compound (a) to react with the compound having an activated carboxy group in a two-phase system of an aqueous phase/an organic phase to obtain the β-lactam compound (b) in the aqueous phase.

2. The production method according to claim 1, wherein the compound having an activated carboxy group is a carboxylic acid chloride compound.

3. The production method according to claim 2, wherein the carboxylic acid chloride compound is 4-ethyl-2,3-dioxopiperazine-1-carbonyl chloride.

4. The production method according to claim 1 or 2, wherein the β-lactam compound (a) is ampicillin or 6-aminopenicillanic acid.

5. The production method according to any one of claims 1 to 3, wherein the liquid (i) contains a basic compound.

6. The production method according to claim 5, wherein the basic compound contained in the liquid (i) is an amine compound.
